# EUROPÄISCHE PATENTSCHRIFT

(11) **EP 1 099 421 B1**
(45) Veröffentlichungstag und Bekanntmachung des Hinweises auf die Patenterteilung: **17.08.2005**
(21) Anmeldenummer: 00124184.3
(22) Anmeldetag: 08.11.2000
(51) Int. Cl.: A61F 2/00

(54) **Flächiges Implantat und Verfahren zu seiner Herstellung**
Prosthetic patch and method of manufacture
Patch prothétique et sa méthode de fabrication

(30) Priorität: 10.11.1999 DE 19954166
(43) Veröffentlichungstag der Anmeldung: 16.05.2001
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Planck, Heinrich, Dr., 72622 Nürtingen (DE); Müller, Erhard, Dr., 70565 Stuttgart (DE); Arnold, Anette, 73734 Esslingen (DE); Schmees, Hans-Gerd, 72827 Wannweil (DE)
(74) Vertreter: Patentanwälte Ruff, Wilhelm, Beier, Dauster & Partner

(56) Entgegenhaltungen:
- EP-A- 0 797 962
- WO-A-99/51163
- CA-A- 2 114 282
- GB-A- 2 222 954
- US-A- 5 569 273
- US-A- 5 593 441

## Beschreibung

Die vorliegende Erfindung betrifft ein flächiges Implantat, ein Verfahren zu seiner Herstellung und seine Verwendung in der Chirurgie.

Körperorgane oder Organteile im Bauchraum von Patienten können Defekte aufweisen, die ihre Organfunktion einschränken und dem Patienten Beschwerden bereiten. Als Beispiele solcher Defekte sind Verschiebungen oder Verlagerungen von Organen sowie Lücken im Gewebe zu nennen. Sie können bedingt sein durch Erkrankungen, altersbedingte Erschlaffung, Muskelschwäche, Bindegewebsschwäche, angeborene Schwächung der Bauchorgane oder ungenügende Narbenbildung nach vorausgegangenen Behandlungen.

Generell kann durch einen chirurgischen Eingriff eine Besserung erreicht werden, jedoch mit dem Nachteil einer hohen Rezidivrate. Es werden daher in der modernen Bauchchirurgie zunehmend künstliche Verstärkungsmaterialien verwendet, die in den Bauchraum des Patienten implantiert werden. Hier spielen besonders Netze aus Polyester, Polypropylen und Polytetrafluorethylen eine Rolle.

Obwohl die Verwendung solcher Netze offensichtlich zu einer deutlichen Verringerung der Rezidivrate geführt hat, sind diese Implantate nicht unproblematisch wegen möglicher Infektionen, Bildung harter Narbenplatten, Verschiebungen oder Fistelbildungen. Gerade im Unterbauchbereich ist wegen der Beinbeweglichkeit eine Versorgung mit möglichst nachgiebigem Implantat und elastische Narbenbildung für rasches Einheilen und Beschwerdefreiheit beim Patienten gefordert.

Die internationale Patentanmeldung WO 99/51163 beschreibt bereits ein Hernienimplantat zur Verwendung in der Chirurgie, wobei dieses Implantat aus zwei Schichten besteht, die miteinander verbunden sind. Diese beiden Schichten zeichnen sich dadurch aus, dass die eine Schicht schneller als die andere abbaubar ist.

Die Erfindung stellt sich die Aufgabe, ein Implantat zum Einsatz bei chirurgischen Eingriffen zur Verfügung zu stellen, das die Schwierigkeiten von Implantaten aus dem Stand der Technik überwindet, einfach und kostengünstig herzustellen ist und in der Praxis bei üblichen chirurgischen Methoden leicht zu handhaben ist.

Die Aufgabe wird gelöst durch ein flächiges Implantat zur Verwendung in der Chirurgie mit einem flexiblen Flächengebilde, das aus mindestens zwei im wesentlichen unabhängig voneinander ausgebildeten textilen Flächenstrukturen gebildet ist, die über die gesamte Fläche des Implantats zu einer Verbundkonstruktion fest miteinander verbunden sind, und wobei die einzelnen textilen Flächenstrukturen in Form von Netzstrukturen ausgebildet sind und die mindestens zwei Netze unterschiedliche Struktur und Bindung aufweisen. Eine solche Verbundstruktur bietet eine hohe Anfangsfestigkeit des Implantats.

Bevorzugt kann das Implantat gemäss der Erfindung im wesentlichen in allen Verbundkomponenten aus Monofilamenten gebildet sein, vorzugsweise ausschliesslich aus Monofilamenten gebildet sein. Die Verwendung von Monofilamenten in Implantatkonstruktionen zeichnet sich gegenüber multifilen Garnen durch eine geringere Infektionsanfälligkeit aus, da Keime keine Aufenthaltsräume wie zwischen Einzelfasern finden.

Mit Vorteil kann ein Monofilament eine Dicke von 10 bis 500 µm, insbesondere von 100 bis 150 µm aufweisen. Gemäss einer Ausführungsform der Erfindung können die Monofilamente der unabhängigen textilen Flächenstrukturen im wesentlichen gleiche Dicken aufweisen. Gemäss einer anderen Ausführungsform der Erfindung können die Monofilamente der unabhängigen textilen Flächenstrukturen unterschiedliche Dicken aufweisen.

Erfindungsgemäss kann das Flächengebilde nach einer textilen Technik, insbesondere Wirken, Stricken, Weben oder Flechten ausgebildet sein. Solche Verfahrenstechniken sind dem Fachmann bekannt, so dass hier auf eine ausführliche Beschreibung verzichtet wird. Gewirkte Flächengebilde sind erfindungsgemäss bevorzugt.

So ist in einer bevorzugten Ausführungsform das Implantat aus Maschenware, insbesondere gewirkter Maschenware gebildet. Auf diese Weise ist eine einfache und kostengünstige Herstellung nach bekannten und in der Praxis bewährten Verfahrenstechniken und mit üblichen Maschinen und Werkzeugen möglich.

Die einzelnen textilen Flächenstrukturen sind in Form von Netzstrukturen, insbesondere gewirkten Netzstrukturen ausgebildet, Maschenwaren zeichnen sich gegenüber anderen Textilkonstruktionen durch eine höhere Flexibilität des Flächengebildes aus, was im Falle der Anwendung in der Medizin wünschenswert ist.

Die Öffnungen oder Poren der Netze können beliebige polygone oder ovale Form aufweisen. Beispielsweise kann die Netzstruktur rautenförmig, gitterförmig, wabenförmig, rund oder langlochförmig ausgebildet sein. Mit Vorteil können Öffnungen mindestens einer Flächenstruktur vorzugsweise im wesentlichen hexagonale Form aufweisen. Ein gewirktes Netz kann beispielsweise eine wabenförmige Struktur aufweisen, wobei hexagonale Poren von aus verwirkten Monofilamenten gebildeten Stegen umgeben sind.

Gemäss der Erfindung können die einzelnen textilen Flächenstrukturen eine Porenstruktur mit Porenweiten bzw. Öffnungsweiten von 0,1 bis 10 mm, insbesondere 0,5 bis 5 mm aufweisen. In einer Ausführungsform der Erfindung können die Porenweiten der einzelnen textilen Flächenstrukturen im wesentlichen gleich gross sein. In einer anderen bevorzugten Ausführungsform der Erfindung können die Porenweiten der einzelnen textilen Flächenstrukturen unterschiedlich gross sein.

Gemäss der Erfindung sind die einzelnen textilen Flächenstrukturen nach voneinander verschiedenen Bindungstechniken hergestellt. Beispielsweise kann in einer bevorzugten Ausführungsform eine textile Fläche durch Wirken nach der Bindungstechnik Atlas 2-reihig ausgebildet sein. In einer anderen bevorzugten Ausführungsform kann eine textile Fläche durch Wirken nach der Bindungstechnik Tüll-Filet ausgebildet sein. Die Herstellung der einzelnen Flächenstrukturen mit unterschiedlichen Bindungen ermöglicht auf einfache Weise die Ausbildung unterschiedlicher Porenformen und Porengrössen.

Mit Vorteil können die textilen Flächenstrukturen durch textile Techniken miteinander verbunden sein. Erfindungsgemäss besonders bevorzugt können die textilen Flächenstrukturen durch Verwirken miteinander verbunden sein. Auf diese Weise können durch Wirken hergestellte Flächenstrukturen einfach und mit denselben Maschinen und Verfahrenstechniken zu einem Verbund kombiniert werden.

Gemäss der Erfindung können die textilen Netzstrukturen so zueinander angeordnet sein, dass ihre Öffnungen nicht fluchtend sind, sich insbesondere etwa hälftig überlappen. Das erfindungsgemässe Implantat kann sich dadurch auszeichnen, dass die textilen Flächenstrukturen, insbesondere Netzstrukturen, sich in beiden Dimensionen der Netzebenen überlappen. Auf diese Weise kann bei gleichem Flächengewicht ein gegen Durchtritt von Stoffen wie Körperflüssigkeiten, Zellen oder Mikroorganismen dichteres Flächengebilde erhalten werden als bei fluchtend ausgerichteten Netzporen. Ferner erleichtert eine engmaschige textile Struktur das Einwachsen des Implantats im Körper und begünstigt somit eine schnelle Heilung.

Solche Netzstrukturkonstruktionen können beispielsweise beim Wirken dadurch hergestellt werden, dass zwei eigenständige Flächengebilde auf seitlich zueinander versetzten Nadeln ausgebildet werden. In einer Ausführungsform können Flächengebilde mit voneinander unterschiedlichen Maschenweiten hergestellt werden. Auf diese Weise können beispielsweise in der lichten Porenweite eines weitmaschigeren Netzes mehrere Maschen eines engmaschigeren Netzes liegen. Besonders vorteilhaft können solche Überlappungsstrukturen durch, insbesondere gleichzeitiges Wirken mit verschiedenen Bindungen hergestellt werden.

Das erfindungsgemässe Implantat kann sich mit Vorteil dadurch auszeichnen, dass es in vivo mindestens teilweise resorbierbar ist. Der Abbau eines bioresorbierbaren Polymers erfolgt im Körper eines Tieres oder eines Menschen durch Stoffwechselvorgänge. An der Umsetzung sind Körper- und Gewebeflüssigkeiten beteiligt. Durch Hydrolyse wird die Polymerkette in kleinere und leichter lösliche Fragmente gespalten. Die Bruchstücke werden ggf. unter Beteiligung enzymatischer Prozesse weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Implantatmaterials beim Patienten ist es wichtig, dass sich während des Abbauvorganges keine schädlichen Metaboliten bilden oder anreichern.

Beim erfindungsgemässen Implantat kann mindestens eine der textilen Flächenstrukturen, insbesondere eine solche mit hexagonalen Öffnungen, im wesentlichen aus nicht resorbierbarem Material gebildet sein und mindestens eine weitere der textilen Flächenstrukturen im wesentlichen aus resorbierbarem Material gebildet sein. Erfindungsgemäss besonders bevorzugt ist eine Ausführungsform, bei der zwei unabhängig voneinander ausgebildete Flächenstrukturen vorgesehen sind, deren eine aus nicht resorbierbarem Material gebildet ist und die zweite aus resorbierbarem Material gebildet ist. Ferner ist es erfindungsgemäss bevorzugt, dass die Flächenstruktur aus nicht resorbierbarem Material hexagonale Öffnungen aufweist.

Mit Vorteil kann zum Verbinden der textilen Flächenstrukturen verwendetes Filamentmaterial aus resorbierbarem Material gebildet sein. Gemäss der Erfindung kann im Implantat resorbierbares und nicht resorbierbares Material im Verhältnis 90 : 10 bis 10 : 90, insbesondere im Verhältnis 30 : 70 bis 70 : 30, bevorzugt im Verhältnis 50 : 50 vorliegen.

Innerhalb von 8 bis 12 Wochen nach Einsetzen des erfindungsgemässen Implantats kommt es infolge der Abbaureaktionen am resorbierbaren Material im Körper des Patienten zu Festigkeitsverlust des Implantats. Bedingt durch den biochemischen Abbau kommt es beim polymeren Filamentmaterial zu Kettenspaltungen und Masseverlust an resorbierbarer Komponente. Dies führt zu fortschreitendem Nachlassen mechanischer Eigenschaften wie beispielsweise Festigkeit und Biegesteifigkeit. Das implantierte Flächengebilde wird zunehmend nachgiebiger, kann sich den örtlichen Gegebenheiten im Bauchraum besser anpassen und Bewegungen des Patienten mitmachen. Mit Vorteil kann im Implantat gemäss der Erfindung die resorbierbare Komponente nach 6 bis 50 Wochen, insbesondere 8 bis 12 Wochen in vivo vollständig degradiert sein.

Mit Vorteil kann durch Abbau des resorbierbaren Materials in vivo die Porengrösse des Implantats vergrössert werden. Auf diese Weise kann eine Versteifung durch Einwachsen von Körperzellen durch Teilabbau der Verbundstruktur mindestens kompensiert werden. Im Laufe der Zeit kann es zu einem Verwachsen des Implantats mit Teilen des Bauchraumes, insbesondere einem Bauchorgan führen. Ein daraus resultierender Verbund von Bauchraum und Implantat trägt zur Stabilisierung des Bauchorgans bei und sichert so den Behandlungserfolg.

Mit fortschreitendem Abbau des resorbierbaren Materials kommt es zu Masseverlust beim Implantat, die sich in einer zunehmend offenporigeren Struktur zeigt. Nach vollständigem Abbau der resorbierbaren Komponente bleibt eine Flächenstruktur aus nicht resorbierbarem Material übrig. Bevorzugt kann die Flächenstruktur aus nicht resorbierbarem Material mit hexagonaler Porenstruktur ausgebildet sein. Eine hexagonale Struktur ist für ein in vivo verbleibendes Implantat besonders vorteilhaft. Auf diese Weise kann durch Wahl der textilen Konstruktion der einzelnen Flächenstrukturen des Implantatverbunds aus nicht resorbierbarem und resorbierbarem Material eine für Einwachsen und Anpassung an physiologische Gegebenheiten optimale Struktur des auf Dauer im Körper des Patienten verbleibenden Implantatteils erhalten werden. Bevorzugt sind die Komponenten der Implantatverbundstruktur so ausgewählt, dass nach Resorption des bioabbaubaren Materials, ein Implantat im Körper verbleibt, das in seinen mechanischen Eigenschaften den natürlichen Eigenschaften der Bauchorgane angepasst ist, bzw. diese wiederherstellt.

Besonders vorteilhaft ist bei der vorliegenden Erfindung eine Ausführungsform, bei der eine Flächenstruktur aus nicht resorbierbarem Material und mindestens eine weitere Flächenstruktur aus resorbierbarem Material zu einer Implantatverbundstruktur ausgebildet sind. Ein weiterer Vorteil der Erfindung liegt in der Verwendung von Monofilamenten zur Ausbildung der textilen Flächenstrukturen. Im Vergleich zu den einzelnen Filamenten in einem Multifilament wie sie aus dem Stand der Technik bekannt sind, weist ein Monofilament eine grössere Dicke auf. Dickere Monofilamente weisen eine höhere Biegesteifigkeit auf, die sich auch auf die Handhabungseigenschaften eines daraus hergestellten textilen Flächengebildes auswirkt. Zum faltenfreien und spannungsfreien Einsetzen von Implantaten im Bauchraum eines Patienten ist eine sichere Handhabbarkeit, also neben Flexibilität eine gewisse Steifigkeit und Festigkeit des Implantats wünschenswert. Dies ist besonders bei streifenförmigen Implantaten von Bedeutung, die um Organe gelegt werden sollen, wie beispielsweise Inkontinenzbänder. Durch Verwendung resorbierbarer Monofilamente in einer erfindungsgemässen Implantatverbundstruktur wird eine solche gewünschte Stabilität erreicht. Während der biochemischen Resorption der abbaubaren Komponente werden mechanische Festigkeit und Steifigkeit des Implantats zunehmend geringer, das Implantat also flexibler, so dass Belastungen des Patienten durch das Implantat ebenfalls abnehmen. Nach Abbau des resorbierbaren Materialanteils verbleibt im Körper des Patienten ein flexibles Netz mit einer nur geringen Menge an Fremdmaterial.

In einer besonderen Ausführungsform kann das Monofilament aus resorbierbarem Material dicker sein als das Monofilament aus nicht resorbierbarem Material. Erfindungsgemäss bevorzugt kann ein resorbierbares Monofilament eine Dicke von 100 bis 250 µm aufweisen. Erfindungsgemäss bevorzugt kann ein nicht resorbierbares Monofilament eine Dicke von 100 bis 250 µm aufweisen. Auf diese Weise kann die nach Resorption des bioabbaubaren Materials verbleibende Menge an Fremdmaterial minimiert werden. Die resorbierbaren und nicht resorbierbaren Monofilamente können gleich dick oder verschieden dick sein.

Erfindungsgemäss kann das nicht resorbierbare Material ein Flächengewicht von bis zu 50 g/m², insbesondere bis zu 40 g/m² aufweisen. Ferner kann das nicht resorbierbare Material eine Festigkeit von 16 bis 50 N/cm aufweisen. Das erfindungsgemässe Implantat kann einen Berstdruck von 100 bis zu 300 kPa aufweisen. Ferner kann das erfindungsgemässe Implantat eine Berstdehnung von 20 bis 50 mm aufweisen.

Mit Vorteil kann sich das Implantat gemäss der Erfindung dadurch auszeichnen, dass seine Dehnbarkeit gemessen in den Richtungen längs, quer und diagonal jeweils um nicht mehr als 50 % unterschiedlich ist, insbesondere weitgehend gleiche Werte aufweist. Das Implantat gemäss der Erfindung kann sich ferner dadurch auszeichnen, dass seine Reisskraft gemessen in den Richtungen längs, quer und diagonal jeweils um nicht mehr als 50 % unterschiedlich ist, insbesondere weitgehend gleiche Werte aufweist.

Beim erfindungsgemässen Implantat kann das nicht resorbierbare Material ausgewählt sein aus der Gruppe bestehend aus Polypropylen, Polytetrafluorethylen, Polytetrafluorethylen-Hexafluorpropylen-Copolymer, Polyethylenterephthalat, Polybutylenterephthalat sowie deren Mischungen, Copolymeren und Terpolymeren. In einer bevorzugten Ausführungsform der Erfindung kann das resorbierbare Material aus monofilen Polylactidfasern gebildet sein. In einer anderen bevorzugten Ausführungsform der Erfindung kann das resorbierbare Material aus monofilen Fasern aus Polylactid-Glykolid-Copolymer gebildet sein.

Beim erfindungsgemässen Implantat kann das resorbierbare Material ausgewählt sein aus der Gruppe bestehend aus Polyglykolid, Polylactid, Polydioxanon, Polyhydroxybuttersäure, Polycaprolacton, Polytrimethylencarbonat, Polytetramethylencarbonat sowie deren Mischungen, Copolymeren und Terpolymeren. In einer bevorzugten Ausführungsform der Erfindung kann das nicht resorbierbare Material aus monofilen Polypropylenfasern gebildet sein.

In einer besonders bevorzugten Ausführungsform kann das erfindungsgemässe Implantat in Form eines Bandes vorliegen.

In Weiterbildung kann im Implantat ein antimikrobiotischer Wirkstoff, wie beispielsweise ein Antibiotikum enthalten sein. Die Verabreichung von Antibiotika dient insbesondere der Vorbeugung von Infektionen. Zur Prophylaxe und Therapie mit Antibiotika finden im Bereich der Chirurgie beispielsweise Cefalosporine wie Cefazolin oder Cefamandol, Netilmicin, Penicilline wie Oxacillin oder Mezlocillin, Tetracyclin, Metronidazol oder Aminoglykoside wie Gentamicin oder Neomycin sowie z. B. Rifampicin Anwendung. Die Fachleute können gemäss den jeweiligen Erfordernissen einen oder mehrere geeignete Wirkstoffe zur Anwendung auswählen. Auch Wachstumsfaktoren können im Implantat enthalten sein.

Die vorliegende Erfindung betrifft ferner ein Verfahren zur Herstellung eines Implantats zur Verwendung in der Chirurgie, das umfasst Ausbilden mindestens zweier voneinander unabhängiger textiler Flächenstrukturen und Verbinden dieser textilen Flächenstrukturen über ihre gesamte Fläche zu einer Verbundkonstruktion in Form eines flexiblen Flächengebildes. Hierbei sind die einzelnen textilen Flächenstrukturen in Form von Netzstrukturen ausgebildet und die mindestens zwei Netze weisen untereschiedliche struktur und Bindung auf.

Bevorzugt können die textilen Flächenstrukturen in Form von Maschenware, insbesondere durch Wirken ausgebildet werden. Erfindungsgemäss können die textilen Flächenstrukturen durch textile Techniken, insbesondere während ihrer gemeinsamen Herstellung durch Verwirken verbunden werden.

Zur Verwendung in der Chirurgie kann das erfindungsgemäss modifizierte Implantat in geeigneter Weise sterilisiert werden. Ein zweckmässiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfasst die Behandlung mit ionisierender Strahlung wie beispielsweise Bestrahlung mit β-Strahlen oder Gammastrahlen, im Bereich von 0,1 Mrad bis 10 Mrad, insbesondere 0,8 Mrad bis 2,5 Mrad.

Des weiteren betrifft die Erfindung auch die Verwendung eines Implantats in der Chirurgie, insbesondere zur Behandlung von Defekten in Körperhöhlen, besonders zum Stützen und Halten von Körperorganen. Als bevorzugtes Beispiel einer solchen Anwendung ist der Einsatz eines streifenförmigen Implantats gemäss der Erfindung als Inkontinenzband zu nennen. Als Harninkontinenzband kann das erfindungsgemässe Implantat zum Unterstützen der weiblichen Harnröhre verwendet werden.

Zu diesem Zweck kann das erfindungsgemässe modifizierte Implantatmaterial auf eine gewünschte Grösse und Form zurechtgeschnitten werden. Mit Vorteil kann das chirurgische Implantat gemäss der Erfindung in zweckmässiger Dimension zugeschnitten gebrauchsfertig in geeigneter Weise verpackt vorliegen. In der Praxis können bevorzugt Abmessungen von 2 bis 5 x 30 bis 50 cm für streifenförmige Implantate oder 30 bis 50 x 30 bis 50 cm für grossflächige Implantate verwendet werden.

Zur Erläuterung sind einige Ausführungsformen der Erfindung als Beispiel in den beigefügten Figuren dargestellt.

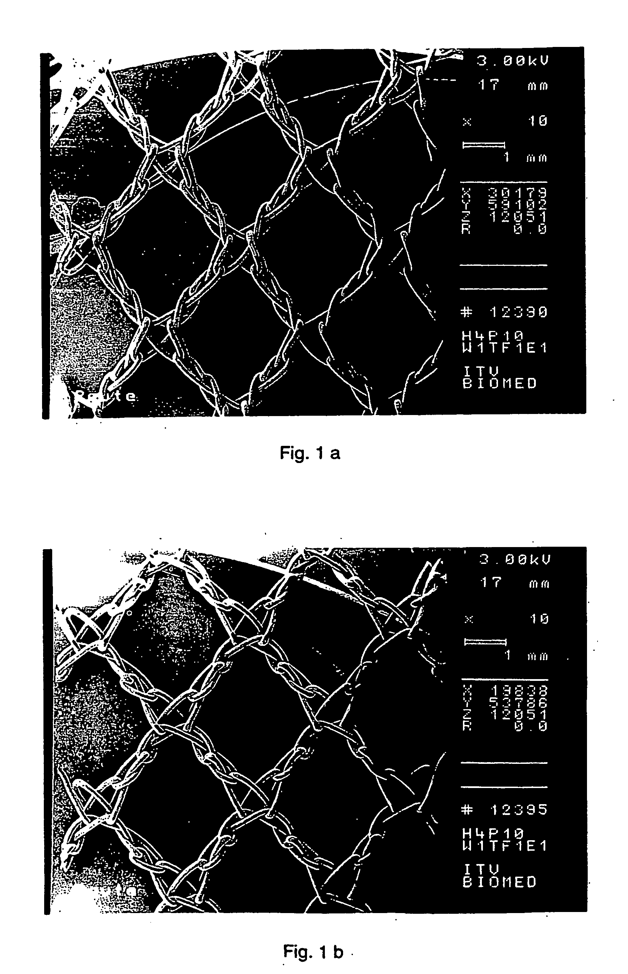

Die Figuren 1a und 1b und zeigen die Vorderseite bzw. Rückseite einer gewirkten Flächenstruktur aus nicht resorbierbarem Polypropylen in Rautenstruktur.

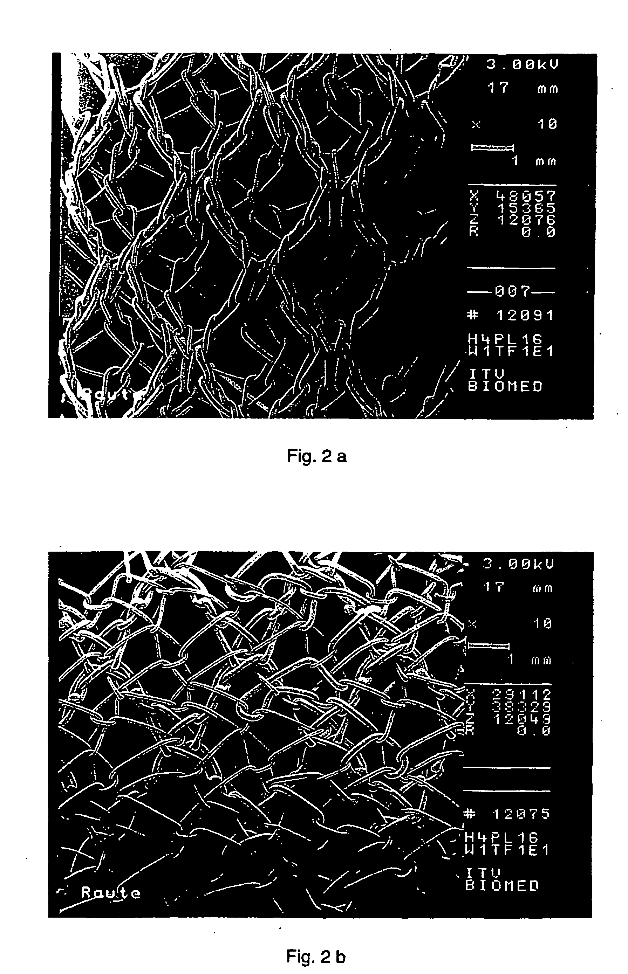

Die Figuren 2a und 2b zeigen die Vorderseite bzw. Rückseite eines teilresorbierbaren Implantats aus nicht resorbierbarem Polypropylen (PP), wie in Figur 1 gezeigt, und damit verwirkter Flächenstruktur aus resorbierbarem Polylaktid (PLLA) in Rautenstruktur. Das Gewirke aus PLLA überlappt die Porenstruktur des Gewirkes aus PP.

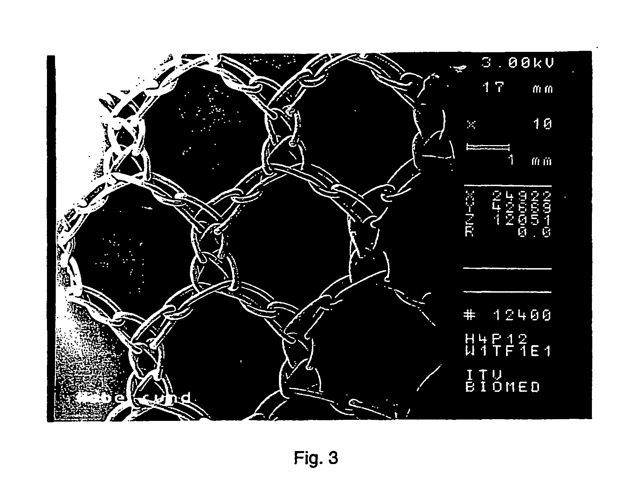

Figur 3 zeigt ein PP-Gewirke in hexagonaler Struktur.

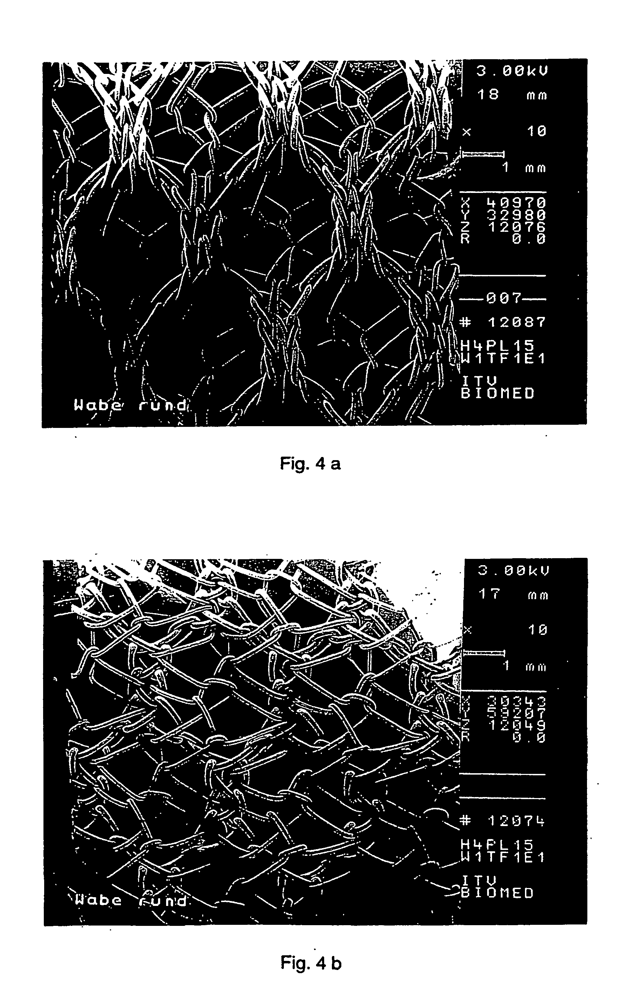

Die Figuren 4a und 4b zeigen die Vorderseite bzw. Rückseite eines teilresorbierbaren Implantats aus nicht resorbierbarem PP, wie in Figur 3 gezeigt, und damit verwirkter Flächenstruktur aus resorbierbarem PLLA in Wabenstruktur.

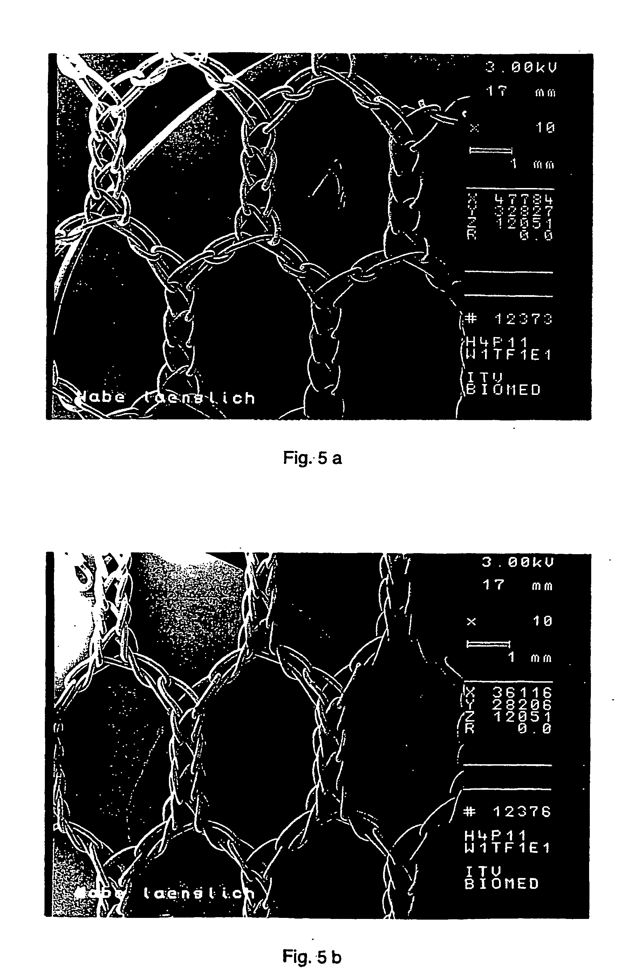

Die Figuren 5a und 5b zeigen die Vorderseite bzw. Rückseite einer gewirkten Flächenstruktur aus nicht resorbierbarem Polypropylen in länglicher Wabenstruktur.

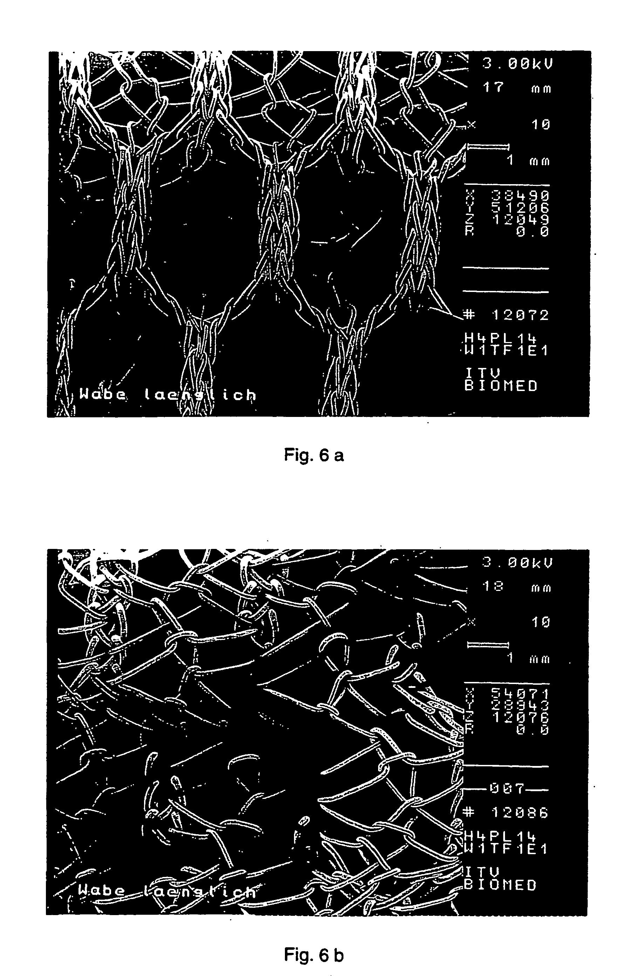

Die Figuren 6a und 6b zeigen die Vorderseite bzw. Rückseite eines teilresorbierbaren Implantats aus nicht resorbierbarem PP, wie in Figur 5 gezeigt, und damit verwirkter Flächenstruktur aus resorbierbarem PLLA in länglicher Wabenstruktur.

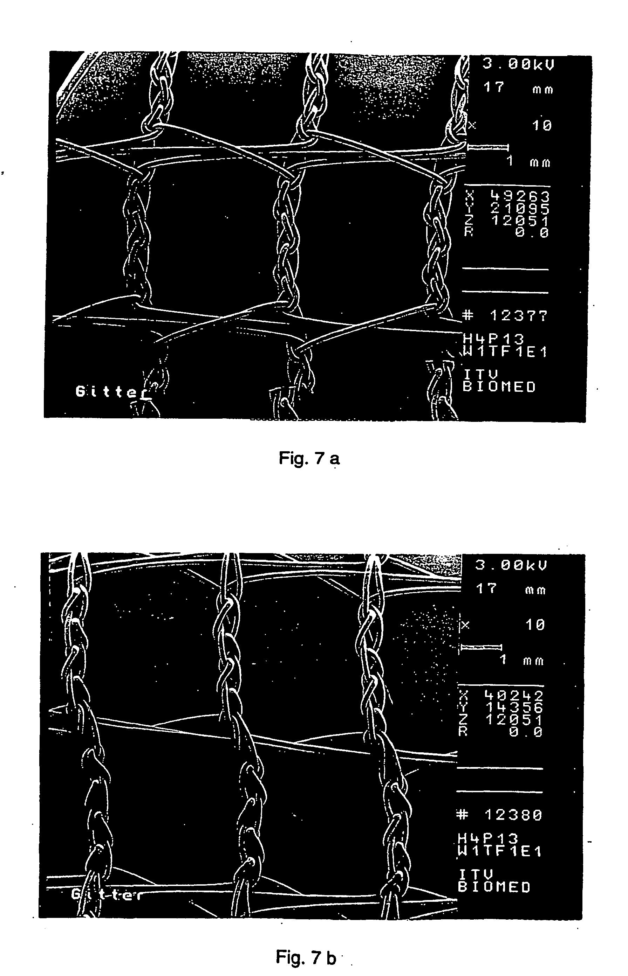

Die Figuren 7a und 7b zeigen die Vorderseite bzw. Rückseite einer gewirkten Flächenstruktur aus nicht resorbierbarem Polypropylen in Gitterstruktur. Die in der Abbildung längs verlaufenden Stränge sind aus verwirkten Fäden gebildet und die Querverbindungen im Gitter sind aus Monofilamenten gebildet.

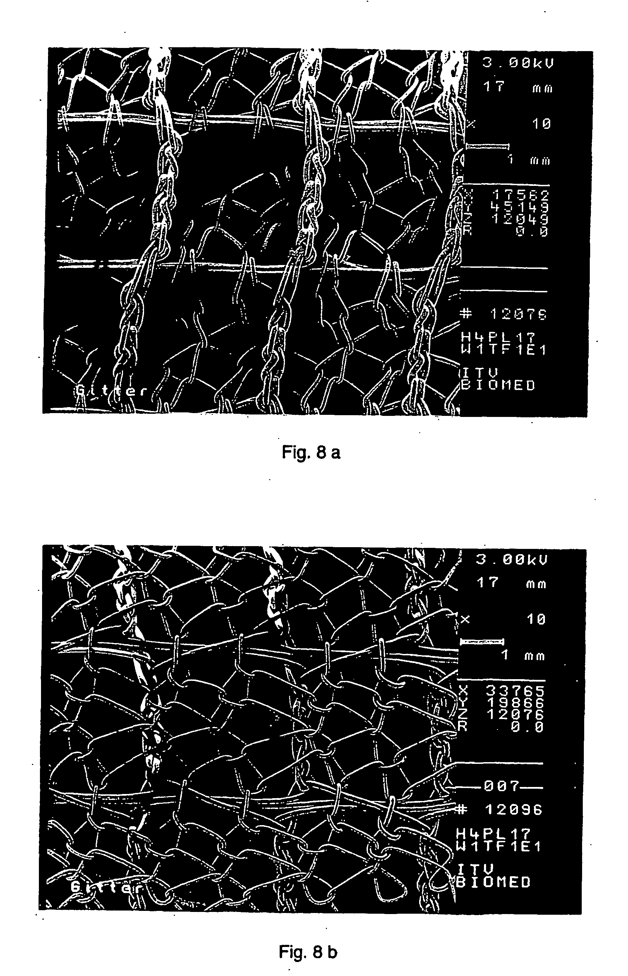

Die Figuren 8a und 8b zeigen die Vorderseite bzw. Rückseite eines teilresorbierbaren Implantats aus nicht resorbierbarem PP, wie in Figur 7 gezeigt, und damit verwirkter Flächenstruktur aus resorbierbarem PLLA. Eine tragende Grobstruktur ist von resorbierbarer Feinstruktur überlagert.

Weitere Merkmale der Erfindung ergeben sich aus der nachfolgenden Beschreibung eines bevorzugten Ausführungsbeispiels. Die in den Unteransprüchen beanspruchten einzelnen Merkmale der Erfindung können jeweils für sich alleine oder zu mehreren bei einer Ausführungsform der Erfindung verwirklicht sein. Das angeführte Beispiel dient nur zur Erläuterung und ist nicht als Einschränkung zu verstehen. Es sind vielmehr für den Fachmann ersichtliche Veränderungen und Modifikationen möglich, ohne den Rahmen der Erfindung zu verlassen.

### Beispiel

Zur Herstellung eines Verbundnetzes aus resorbierbarem und nichtresorbierbarem bioverträglichem Polymermaterial werden Monofilamente aus Polylactid (PLLA) und Polypropylen (PP) auf einer Kettenwirkmaschine verarbeitet. Für das resorbierbare Flächengebilde aus PLLA wird nach der Technik Atlas 2reihig gearbeitet. Die Kettaufstellung dieser Bindung ist: 2-0/4-6/8-10/6-4/2-0/4-6/8-10/6-4, so dass diese Legeschiene auf den Nadeln 2, 4, 6, 8, 10 usw. arbeitet. Für das nicht resorbierbare Flächengebilde aus PP wird nach der Technik Tüll-Filet gearbeitet. Die Kettaufstellung dieser Bindung ist: eine Legeschiene 2-0/4-6/2-0/4-6/8-10/6-4/8-10/6-4, zweite Legeschiene 8-10/6-4/8-10/6-4/2-0/4-6/2-0/4-6, so dass beide Legeschienen auf den Nadeln 1, 3, 5, 7, 9 usw. arbeiten. Die beiden verschiedenen Bindungen, Atlas 2-reihig und Tüll-Filet werden nicht auf den gleichen Nadeln gearbeitet, sondern um eine Nadel seitlich versetzt. Dadurch bildet das resorbierbare Gewirk eine Maschenstruktur mit kleineren Porenweiten im Vergleich zur Maschenstruktur des nicht resorbierbaren Gewirks. Zwischen den Stegen der Wabenstruktur aus nicht resorbierbarem Material befinden sich jeweils mehrere Maschen aus resorbierbarem Material. Die beiden gewirkten Flächengebilde werden durch Unterlegungen miteinander verbunden. Durch Unterlegung ist in vivo ein Abbau des resorbierbaren Gewirks ohne Wechselwirkung auf das nicht resorbierbare Gewirk möglich.

Das Verbundnetz kann grossflächig hergestellt und auf die gewünschte Grösse zugeschnitten werden. Für ein Harninkontinenzband eignen sich Streifen mit einer Länge von 30 bis 50 cm und einer Breite von 2 bis 5 cm.

## Patentansprüche

1. Flächiges Implantat zur Verwendung in der Chirurgie mit einem flexiblen Flächengebilde, das aus mindestens zwei im wesentlichen unabhängig voneinander ausgebildeten textilen Flächenstrukturen gebildet ist, die über die gesamte Fläche des Implantats zu einer Verbundkonstruktion fest miteinander verbunden sind, und wobei die einzelnen textilen Flächenstrukturen in Form von Netzstrukturen ausgebildet sind, **dadurch gekennzeichnet, dass** die mindestens zwei Netze unterschiedliche Struktur und Bindung aufweisen.

2. Implantat nach Anspruch 1, **dadurch gekennzeichnet, dass** es im wesentlichen in allen Verbundkomponenten aus Monofilamenten gebildet ist, vorzugsweise ausschliesslich aus Monofilamenten gebildet ist.

3. Implantat nach einem der vorhergehenden Ansprüche, **dadurch gekennzeichnet, dass** ein Monofilament eine Dicke von 10 bis 500 µm, insbesondere 100 bis 150 µm aufweist.

4. Implantat nach einem der vorhergehenden Ansprüche, **dadurch gekennzeichnet, dass** die Netzstrukturen in Form von gewirkten Netzstrukturen ausgebildet sind.

5. Implantat nach einem der vorhergehenden Ansprüche, **dadurch gekennzeichnet, dass** mindestens eine Flächenstruktur Öffnungen aufweist, die vorzugsweise im wesentlichen hexagonale Form aufweisen.

6. Implantat nach einem der vorhergehenden Ansprüche, **dadurch gekennzeichnet, dass** die einzelnen textilen Flächenstrukturen eine Porenstruktur mit Porenweiten von 0,1 bis 10 mm, insbesondere 0,5 bis 5 mm aufweisen.

7. Implantat nach einem der vorhergehenden Ansprüche, **dadurch gekennzeichnet, dass** die textilen Flächenstrukturen durch Verwirken miteinander verbunden sind.

8. Implantat nach einem der vorhergehenden Ansprüche, **dadurch gekennzeichnet, dass** die textilen Netzstrukturen so zueinander angeordnet sind, dass ihre Öffnungen nicht fluchtend sind, sich insbesondere etwa hälftig überlappen.

9. Implantat nach einem der vorhergehenden Ansprüche, **dadurch gekennzeichnet, dass** es in vivo mindestens teilweise resorbierbar ist.

10. Implantat nach einem der vorhergehenden Ansprüche, **dadurch gekennzeichnet, dass** mindestens eine der textilen Flächenstrukturen, insbesondere eine solche mit hexagonalen Öffnungen, im wesentlichen aus nicht resorbierbarem Material gebildet ist und mindestens eine weitere der textilen Flächenstrukturen im wesentlichen aus resorbierbarem Material gebildet ist.

11. Implantat nach einem der vorhergehenden Ansprüche, **dadurch gekennzeichnet, dass** resorbierbares und nicht resorbierbares Material im Verhältnis 90 : 10 bis 10 : 90, insbesondere im Verhältnis 30 : 70 bis 70 : 30, bevorzugt 50 : 50 vorliegt.

12. Implantat nach einem der vorhergehenden Ansprüche, **dadurch gekennzeichnet, dass** seine Dehnbarkeit gemessen in den Richtungen längs, quer und diagonal jeweils um nicht mehr als 50 % unterschiedlich ist, insbesondere weitgehend gleiche Werte aufweist.

13. Implantat nach einem der vorhergehenden Ansprüche, **dadurch gekennzeichnet, dass** seine Reisskraft gemessen in den Richtungen längs, quer und diagonal, jeweils um nicht mehr als 50 % unterschiedlich ist, insbesondere weitgehend gleiche Werte aufweist.

14. Implantat nach einem der Ansprüche 10 bis 13, **dadurch gekennzeichnet, dass** das nicht resorbierbare Material ausgewählt ist aus der Gruppe bestehend aus Polypropylen, Polytetrafluorethylen, Polytetrafluorethylen-Hexafluorpropylen-Copolymer, Polyethylenterephthalat, Polybutylenterephthalat sowie deren Mischungen, Copolymeren und Terpolymeren.

15. Implantat nach einem der Ansprüche 10 bis 14, durch **gekennzeichnet**, dass das resorbierbare Material ausgewählt ist aus der Gruppe bestehend aus Polyglykolid, Polylactid, Polydioxanon, Polyhydroxybuttersäure, Polycaprolacton Polytrimethylencarbonat, Polytetramethylencarbonat sowie deren Mischungen, Copolymeren und Terpolymeren.

16. Implantat nach einem der vorhergehenden Ansprüche, **dadurch gekennzeichnet, dass** es in Form eines Bandes vorliegt.

17. Verfahren zur Herstellung eines Implantats durch Ausbilden mindestens zweier im wesentlichen voneinander unabhängiger textiler Flächenstrukturen und Verbinden dieser textilen Flächenstrukturen über ihre gesamte Fläche zu einer Verbundkonstruktion in Form eines flexiblen Flächengebildes, wobei die einzelnen textilen Flächenstrukturen in Form von Netzstrukturen ausgebildet sind, **dadurch** gekennzeichet, dass die mindestens zwei Netze unterschiedliche Struktur und Bindung aufweisen.

18. Verfahren nach Anspruch 17, **dadurch gekennzeichnet, dass** die textilen Flächenstrukturen in Form von Maschenware, insbesondere durch Wirken ausgebildet werden.

19. Verfahren nach Anspruch 17 oder 18, **dadurch gekennzeichnet, dass** die textilen Flächenstrukturen durch textile Techniken, insbesondere während ihrer gemeinsamen Herstellung durch Verwirken verbunden werden.

20. Verwendung eines flexiblen Flächengebildes, das aus mindestens zwei unabhängig voneinander ausgebildeten textilen Flächenstrukturen gebildet ist, die über die gesamte Fläche zu einer Verbundkonstruktion fest miteinander verbunden sind, und wobei die einzelnen textilen Flächenstrukturen in Form von Netzstrukturen ausgebildet sind und die mindestens zwei Netze unterschiedliche Struktur und Bindung aufweisen, zur Herstellung eines flächigen Implantats in der Chirurgie zum Stützen und Halten von Körperorgarnen, insbesondere als Harninkontinenzband zum Unterstützen der weiblichen Harnröhre.

## Claims

1. Prosthetic patch for use in surgery, with a flexible fabric formed from at least two textile fabric structures which are constructed substantially independently of one another and are firmly connected to one another across the entire surface of the patch so as to form a composite construction, the individual textile fabric structures being in the form of mesh structures, **characterized in that** the at least two meshes have a different structure and weave.

2. Patch according to Claim 1, **characterized in that** it is formed from monofilaments in substantially all the composite components, preferably exclusively from monofilaments.

3. Patch according to one of the preceding claims, **characterized in that** a monofilament has a thickness of 10 to 500 µm, in particular of 100 to 150 µm.

4. Patch according to one of the preceding claims, **characterized in that** the mesh structures are in the form of knitted mesh structures.

5. Patch according to one of the preceding claims, **characterized in that** at least one fabric structure has openings which preferably have a substantially hexagonal shape.

6. Patch according to one of the preceding claims, **characterized in that** the individual textile fabric structures have a pore structure with pore widths of 0.1 to 10 mm, in particular 0.5 to 5 mm.

7. Patch according to one of the preceding claims, **characterized in that** the textile fabric structures are connected to one another by knitting.

8. Patch according to one of the preceding claims, **characterized in that** the textile mesh structures are arranged relative to one another in such a way that their openings are not aligned, in particular in such a way that their openings overlap by roughly half.

9. Patch according to one of the preceding claims, **characterized in that** it is at least partially absorbable in vivo.

10. Patch according to one of the preceding claims, **characterized in that** at least one of the textile fabric structures, in particular one with hexagonal openings, is formed substantially from non-absorbable material, and at least one other of the textile fabric structures is formed substantially from absorbable material.

11. Patch according to one of the preceding claims, **characterized in that** absorbable and non-absorbable materials are present in a ratio of 90:10 to 10:90, in particular in a ratio of 30:70 to 70:30, preferably 50:50.

12. Patch according to one of the preceding claims, **characterized in that** its extensibility, measured in the longitudinal, transverse and diagonal directions, in each case does not differ by more than 50%, and in particular has largely identical values.

13. Patch according to one of the preceding claims, **characterized in that** its tear strength, measured in the longitudinal, transverse and diagonal directions, in each case does not differ by more than 50%, and in particular has largely identical values.

14. Patch according to one of Claims 10 to 13, **characterized in that** the non-absorbable material is chosen from the group comprising polypropylene, polytetrafluoroethylene, polytetrafluoroethylene-hexafluoropropylene copolymer, polyethylene terephthalate, polybutylene terephthalate, and their mixtures, copolymers and terpolymers.

15. Patch according to one of Claims 10 to 14, **characterized in that** the absorbable material is chosen from the group comprising polyglycolide, polylactide, polydioxanone, polyhydroxybutyric acid, polycaprolactone, polytrimethylene carbonate, polytetramethylene carbonate, and their mixtures, copolymers and terpolymers.

16. Patch according to one of the preceding claims, **characterized in that** it is in the form of a band.

17. Method for manufacture of a patch by forming at least two substantially independent textile fabric structures and connecting these textile fabric structures across their entire surface to form a composite construction in the form of a flexible fabric, the individual textile fabric structures being in the form of mesh structures, **characterized in that** the at least two meshes have a different structure and weave.

18. Method according to Claim 17, **characterized in that** the textile fabric structures are in the form of knitwear, in particular produced by knitting.

19. Method according to Claim 17 or 18, **characterized in that** the textile fabric structures are connected by textile techniques, in particular by knitting together during their joint manufacture.

20. Use of a flexible fabric formed from at least two textile fabric structures which are constructed independently of one another and are firmly connected to one another across the entire surface to form a composite construction, the individual textile fabric structures being in the form of mesh structures, and the at least two meshes having a different structure and weave, for manufacture of a prosthetic patch in surgery for supporting and holding organs of the body, in particular as a urinary incontinence band for supporting the female urethra.

## Revendications

1. Implant plat destiné à être utilisé en chirurgie, qui présente un produit plat flexible qui est formé d'au moins deux structures textiles plates réalisées indépendamment l'une de l'autre qui sont reliées solidairement l'une à l'autre sur toute la surface de l'implant pour former une structure composite, les structures textiles plates individuelles étant configurées sous la forme de structures en treillis, **caractérisé en ce qu'**au moins deux treillis présentent des structures et des liaisons différentes.

2. Implant selon la revendication 1, **caractérisé en ce que** dans tous les composants du composite, il est formé essentiellement de monofilaments et de préférence exclusivement de monofilaments.

3. Implant selon l'une des revendications précédentes, **caractérisé en ce qu'**un monofilament présente une épaisseur de 10 à 500 µm et en particulier de 100 à 150 µm.

4. Implant selon l'une des revendications précédentes, **caractérisé en ce que** les structures en treillis sont configurées sous la forme de structures en treillis tricoté.

5. Implant selon l'une des revendications précédentes, **caractérisé en ce qu'**au moins une structure plate présente des ouvertures qui ont de préférence une forme essentiellement hexagonale.

6. Implant selon l'une des revendications précédentes, **caractérisé en ce que** les structures textiles plates individuelles présentent une structure poreuse dont les pores ont une taille comprise entre 0,1 et 10 mm et en particulier entre 0,5 et 5 mm.

7. Implant selon l'une des revendications précédentes, **caractérisé en ce que** les structures textiles plates sont reliées les unes aux autres par tricotage.

8. Implant selon l'une des revendications précédentes, **caractérisé en ce que** les structures textiles en treillis sont disposées l'une par rapport à l'autre de telle sorte que leurs ouvertures ne soient pas alignées et qu'en particulier elles se superposent sensiblement pour moitié.

9. Implant selon l'une des revendications précédentes, **caractérisé en ce qu'**il est au moins en partie résorbable in vivo.

10. Implant selon l'une des revendications précédentes, **caractérisé en ce qu'**au moins l'une des structures textiles plates et en particulier une structure textile plate qui présente des ouvertures hexagonales est formée essentiellement d'un matériau non résorbable et **en ce qu'**au moins une autre des structures textiles plates est formée essentiellement d'un matériau résorbable.

11. Implant selon l'une des revendications précédentes, **caractérisé en ce que** le matériau résorbable et le matériau non résorbable sont présents dans un rapport de 90 : 10 à 10 : 90, en particulier dans le rapport de 30 : 70 à 70 : 30 et de préférence de 50 : 50.

12. Implant selon l'une des revendications précédentes, **caractérisé en ce que** ses expansibilités mesurées dans le sens de la longueur, le sens transversal et le sens diagonal ne diffèrent pas les unes des autres de plus de 50 % et présentent en particulier des valeurs largement identiques.

13. Implant selon l'une des revendications précédentes, **caractérisé en ce que** ses résistances à la déchirure mesurées dans le sens de la longueur, le sens transversal et le sens diagonal ne diffèrent pas les unes des autres de plus 50 % et présentent en particulier des valeurs largement identiques.

14. Implant selon l'une des revendications 10 à 13, **caractérisé en ce que** le matériau non résorbable est sélectionné dans l'ensemble constitué du polypropylène, du polytétrafluoroéthylène, d'un copolymère de polytétrafluoroéthylène et d'hexafluoropropylène, du poly(téréphtalate d'éthylène), du poly(téréphtalate de butylène) ainsi que de leurs mélanges, de leurs copolymères et terpolymères.

15. Implant selon l'une des revendications 10 à 14, **caractérisé en ce que** le matériau résorbable est sélectionné dans l'ensemble constitué des polyglycolides, des polylactides, des polydioxanones, du poly(acide hydroxybutyrique), du polycaprolactone, du poly(carbonate de triméthylène), du poly(carbonate de tétraméthylène) ainsi que de leurs mélanges, copolymères et terpolymères.

16. Implant selon l'une des revendications précédentes, **caractérisé en ce qu'**il est présent sous la forme d'une bande.

17. Procédé de fabrication d'un implant par formation d'au moins deux structures textiles plates essentiellement indépendantes l'une de l'autre et liaison de ces structures textiles plates sur toute leur surface pour former une structure composite qui présente la forme d'un produit plat flexible, les structures textiles plates individuelles présentant la forme de structures en treillis, **caractérisé en ce que** les au moins deux treillis présentent des structures et des liaisons différentes.

18. Procédé selon la revendication 17, **caractérisé en ce que** les structures textiles plates sont configurées sous la forme de tissus maillés obtenus en particulier par tricotage.

19. Procédé selon les revendications 17 ou 18, **caractérisé en ce que** les structures textiles plates sont reliées par des techniques textiles, en particulier par tricotage pendant leur fabrication commune.

20. Utilisation d'un produit plat flexible qui est formé d'au moins deux structures textiles plates réalisées indépendamment l'une de l'autre qui sont solidarisées l'une de l'autre sur toute leur surface pour fournir une structure composite, les structures textiles plates individuelles présentant la forme de structures en treillis et les au moins deux treillis présentant des structures et des liaisons différentes, en vue de la fabrication d'un implant plat en chirurgie pour le support et le maintien d'organes corporels, en particulier en tant que bande de continence urinaire en vue du support des canaux urinaires féminins.
